# FASCICULE DE BREVET EUROPEEN

(11) **EP 0 569 287 B1**
(45) Date de publication et mention de la délivrance du brevet: **10.07.1996**
(21) Numéro de dépôt: 93401149.5
(22) Date de dépôt: 04.05.1993
(51) Int. Cl.: A61F 5/453

(54) **Applicateur pour cathéter externe de collecte urinaire, et dispositif de collecte urinaire comportant ledit applicateur**
Hilfsvorrichtung zum Anbringen eines externen Urinsammel- katheters und Urinsammelvorrichtung mit der Hilfsvorrichtung
Applicator for external urine collection catheter and urinary collection device comprising said applicator

(30) Priorité: 05.05.1992 FR 9205516
(43) Date de publication de la demande: 10.11.1993
(73) Titulaire: SYNTHELABO, F-92350 Le Plessis Robinson (FR)
(72) Inventeur: Di Cristo, Marin, F-06200 Nice (FR)
(74) Mandataire: Bonnetat, Christian

(56) Documents cités:
- EP-A- 0 284 224
- WO-A-88/02624
- US-A- 4 589 874

## Description

La présente invention concerne un applicateur pour un cathéter externe de collecte urinaire pour individus de sexe masculin, et un dispositif de collecte urinaire comportant ledit applicateur.

On connaît des dispositifs de collecte urinaire comportant essentiellement un cathéter externe comprenant un manchon souple destiné à être appliqué de façon étanche sur le pénis d'un patient, lequel manchon est raccordé à une partie de réception du gland, sensiblement en forme de calotte sphérique, elle-même raccordée à un tube d'évacuation de l'urine, ledit manchon étant enroulé sur lui-même préalablement à son application. Une difficulté consiste en la mise en place d'un tel cathéter du fait du manque de rigidité du pénis généralement constatée chez le patient au moment de l'application du cathéter. Pour mettre en place ce dernier, il est donc nécessaire d'utiliser un applicateur. Un ensemble cathéter-applicateur est décrit, notamment, dans le brevet US-A-4 589 874. Dans ce cas, l'applicateur est constitué d'un corps tubulaire destiné à entourer partiellement la partie de réception du gland, et présente un bourrelet derrière lequel le manchon est maintenu en position enroulée, en permettant d'éviter un déroulement involontaire dudit manchon. Par ailleurs, le corps présente des évidements qui, lors de la mise en place du cathéter, servent d'ouvertures pour les doigts de l'opérateur pour saisir la partie de réception du gland et l'extrémité de ce dernier, alors que, de l'autre main, l'opérateur déroule le manchon. Cependant, dans ces circonstances, on constate souvent une rétraction du pénis telle qu'elle empêche la mise en place d'un tel cathéter malgré l'emploi d'un applicateur, notamment comme celui décrit dans le brevet US-A-4 589 874.

La présente invention a pour but d'éviter cet inconvénient, et concerne un applicateur pour un cathéter externe de collecte urinaire, adapté pour maintenir, de façon positive, le pénis, et l'empêcher de se rétracter pendant la mise en place du cathéter.

A cet effet, l'applicateur pour cathéter externe de collecte urinaire, ledit cathéter comportant un manchon souple destiné à être appliqué de façon étanche sur le pénis d'un patient, lequel manchon est raccordé à une partie de réception du gland, de forme tubulaire, elle-même raccordée à un tube d'évacuation de l'urine, ledit manchon étant enroulé sur lui-même préalablement à son application, et ledit applicateur comportant un corps tubulaire destiné à entourer au moins partiellement ladite partie de réception du gland, est remarquable, selon l'invention, en ce que ledit corps comprend au moins deux mors commandables dont une extrémité de chacun d'entre eux est solidaire d'un support annulaire, et dont l'autre extrémité libre présente une saillie radialement interne destinée à venir s'accrocher derrière le gland et à maintenir celui-ci pendant le déroulement du manchon souple sur le pénis.

Ainsi, en saisissant le gland par les mors, l'opérateur peut tirer la verge de façon à provoquer son extension pour de ce fait faciliter le déroulement du manchon sur celle-ci.

Avantageusement, l'applicateur comprend trois mors, dont un mors central destiné à venir s'appliquer derrière la face supérieure du gland, et deux mors latéraux disposés de part et d'autre, respectivement, du mors central.

De préférence, les extrémités libres des deux mors latéraux sont biseautées de façon correspondant à la forme anatomique des parties latérales du gland derrière lesquelles ils doivent venir s'accrocher.

En particulier, la saillie radialement interne du mors central peut être intérieurement arrondie.

Par ailleurs, selon un premier exemple de réalisation avantageux de l'invention, le corps dudit applicateur présente une forme générale cylindrique, et des pattes de liaison sont prévues entre chacun desdits mors, reliant ledit support annulaire à une bague munie d'une gorge externe de réception du manchon en position enroulée.

Dans ce cas, avantageusement, les pattes de liaison présentent chacune, au voisinage de ladite bague, un épaississement interne dans le prolongement des saillies radialement internes des mors, de façon que l'ensemble saillies-épaississements des pattes présente une forme correspondant à la forme anatomique du gland.

Selon un autre exemple de réalisation de l'invention, le corps dudit applicateur présente une forme générale de calotte sphérique, et, sur la face externe de chacun desdits mors, est prévue une partie de préhension s'effilant en forme de coin vers l'extrémité libre du mors.

Dans ce dernier cas, de préférence, l'applicateur comprend, au voisinage des extrémités libres des mors, une bague, qui peut être désolidarisée desdits mors lorsque ceux-ci sont pressés les uns vers les autres, et qui comporte une gorge de réception du manchon en position enroulée.

Avantageusement, ladite bague comprend un rebord d'appui du manchon facilitant son déroulement.

Les figures de dessin annexé feront bien comprendre comment l'invention peut être réalisée. Sur ces figures, des références identiques désignent des éléments semblables.

La figure 1 est une vue en perspective éclatée d'un premier exemple de réalisation du dispositif de collecte urinaire selon l'invention.

Les figures 2 et 3 sont deux vues en perspective, tournées l'une par rapport à l'autre de 180°, de l'applicateur du dispositif de collecte urinaire de la figure 1.

Les figures 4a-4d illustrent différentes étapes de la mise en place du cathéter externe de collecte urinaire selon les figures 1 à 3, sur le pénis du patient.

La figure 5 est une vue en perspective éclatée d'un second exemple de réalisation du dispositif de collecte urinaire selon l'invention.

La figure 6 est une vue en perspective de l'applicateur du dispositif de collecte urinaire de la figure 5 sous un angle différent.

La figure 7 est une vue en coupe du dispositif de la figure 5 avec l'applicateur en place.

Les figures 8a-8d illustrent différentes étapes de la mise en place du cathéter externe de collecte urinaire selon les figures 5 à 7, sur le pénis d'un patient.

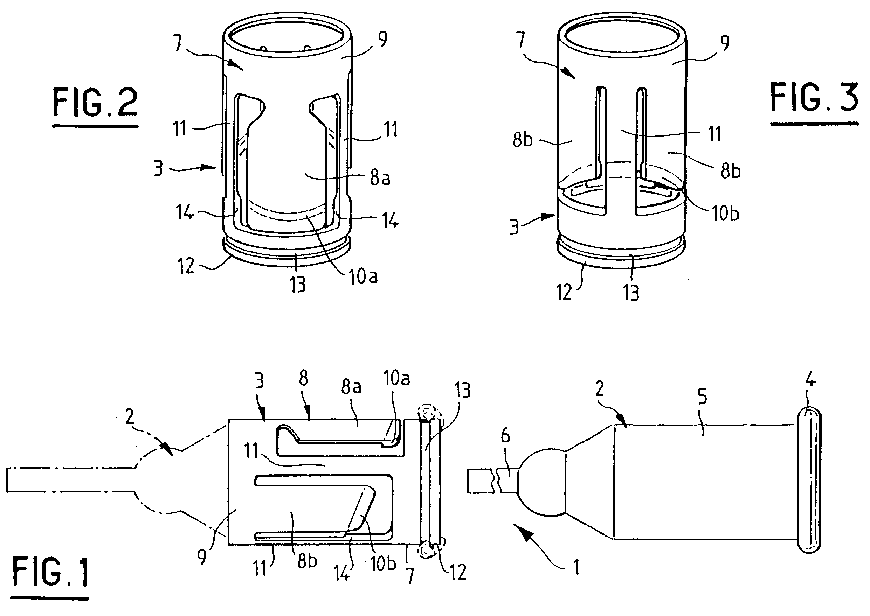

Comme on le voit sur les figures 1 à 3, un premier exemple de réalisation du dispositif 1 de collecte urinaire selon l'invention comporte un cathéter externe 2 et un applicateur 3. Pour des raisons de clarté du dessin, on a représenté, sur la figure 1, le cathéter 2 (en traits pleins) séparé de l'applicateur 3, et le cathéter 2 (en traits mixtes) alors que l'applicateur 3 est installé sur celui-ci. Le cathéter 2 comprend un manchon souple 4, montré en position enroulée sur lui-même sur la figure 1, et destiné à être appliqué sur le pénis d'un patient, lequel manchon 4 est raccordé à une partie de réception 5 du gland, de forme cylindrique, elle-même raccordée à un tube 6 d'évacuation de l'urine (pouvant être relié à de moyens de collecte de l'urine non montrés).

Par ailleurs, l'applicateur 3 comporte un corps tubulaire 7 de forme cylindrique destiné à entourer la partie de réception 5 du gland, et comportant un jeu 8 de trois mors commandables 8a, 8b, 8b. Parmi ces derniers, un mors central 8a est destiné à venir s'appliquer derrière la face supérieure du gland, et les deux mors latéraux 8b,8b, disposés de part et d'autre, respectivement, du mors central 8a, sont destinés à venir s'accrocher derrière les parties latérales du gland. Une extrémité de chacun des mors 8a, 8b, 8b est solidaire d'un support annulaire 9, tandis que l'autre extrémité libre présente une saillie 10a, 10b, 10b radialement interne, chacune desdites saillies étant destinée à venir s'accrocher derrière le gland et à maintenir celui-ci pendant le déroulement du manchon 4 sur le pénis. A cet effet, les extrémités libres des deux mors latéraux 8b, 8b sont biseautées de façon correspondant à la forme anatomique des parties latérales du gland, tandis que la saillie radialement interne 10a du mors central 8a est intérieurement arrondie.

Par ailleurs, des pattes de liaison 11 sont prévues entre chacun des mors 8a, 8b, 8b, reliant le support annulaire 9 à une bague 12 munie d'une gorge externe 13 de réception du manchon 4 en position enroulée (en traits mixtes sur la figure 1). Les pattes de liaison 11 présentent chacune, au voisinage de la bague 12, un épaississement interne 14 dans le prolongement des saillies radialement internes 10a, 10b, 10b des mors 8a, 8b, 8b, de façon que l'ensemble saillies-épaississements des pattes présente une forme correspondant à la forme anatomique du gland.

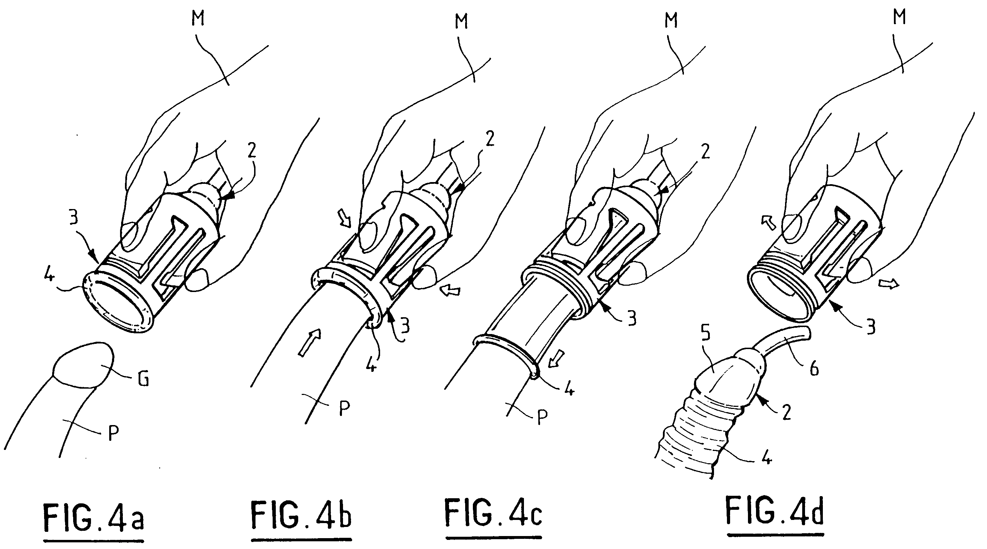

Les figures 4a-4d montrent la mise en place du cathéter externe de collecte urinaire 2 sur le pénis P d'un patient. Le dispositif 1 étant dans la position montée, montrée en traits mixtes sur la figure 1, c'est-à-dire avec l'applicateur 3 en place sur le cathéter 2 et le manchon 4 de ce dernier logé dans la gorge 13 de la bague 12, la main M de l'opérateur saisit, par trois doigts, les trois mors 8a, 8b, 8b de l'applicateur 3 (figure 4a) et, lorsque le gland G du pénis P est en position dans la partie de réception 5 du cathéter 2 (figure 4b), l'opérateur, en pressant sur les mors, et grâce aux saillies radialement internes 10a, 10b, 10b de ceux-ci, peut venir accrocher l'applicateur derrière le gland et, ainsi, tirer sur la verge du patient, en évitant la rétraction de cette dernière, tout en déroulant le manchon 4 sur le pénis (figure 4c). Lorsque le manchon 4 est totalement déroulé (figure 4d), le cathéter 2 est en place, et l'opérateur, en relâchant la pression sur les mors, peut retirer l'applicateur 3.

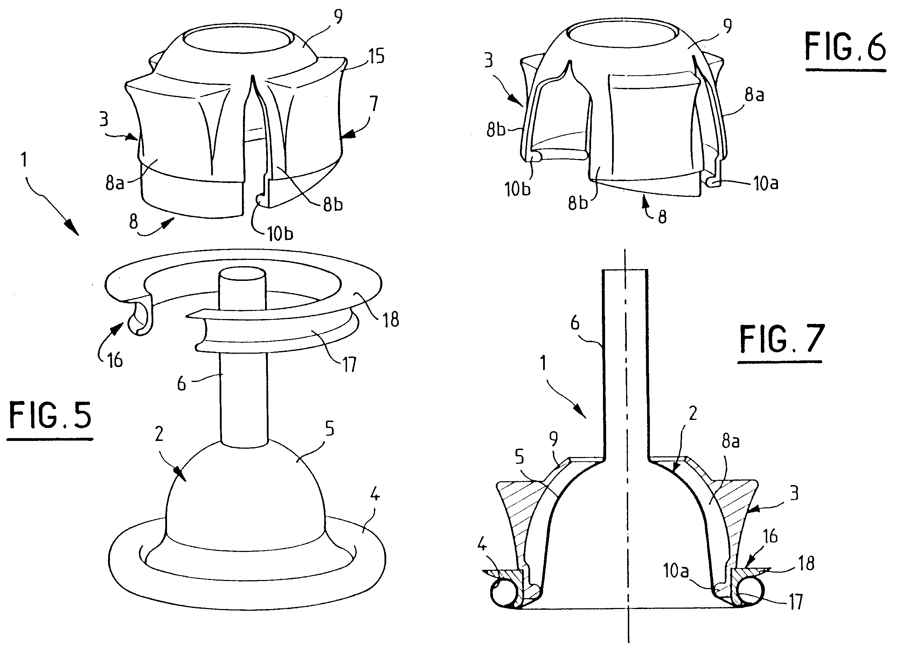

Le second exemple de réalisation du dispositif 1 de collecte urinaire, montré sur les figures 5 à 7, comporte, comme dans le premier exemple, un cathéter externe 2 et un applicateur 3. Le cathéter 2 comprend encore un manchon souple 4, raccordé à une partie de réception 5 du gland, de forme tronconique, elle-même raccordée à un tube 6 d'évacuation de l'urine.

Par ailleurs, l'applicateur 3 comporte un corps 7 sensiblement en forme de calotte sphérique, destiné à entourer la partie de réception 5 du gland, et comportant un jeu 8 de trois mors commandables 8a, 8b, 8b. Comme dans l'exemple de réalisation précédent, un mors central 8a est destiné à venir s'appliquer derrière la face supérieure du gland, et les deux mors latéraux 8b, 8b, disposés de part et d'autre, respectivement, du mors central 8a, sont destinés à venir s'accrocher derrière les paries latérales du gland. Une extrémité de chacun des mors 8a, 8b, 8b est solidaire d'un support annulaire 9, tandis que l'autre extrémité libre présente une saillie 10a, 10b, 10b radialement interne, chacune desdites saillies étant destinée à venir s'accrocher derrière le gland et à maintenir celui-ci pendant le déroulement du manchon 4 sur le pénis. A cet effet, les extrémités libres des deux mors latéraux 8b, 8b sont biseautées de façon correspondant à la forme anatomique des parties latérales du gland, tandis que la saillie radialement interne 10a du mors central 8a est intérieurement arrondie.

De plus, sur la face externe de chacun des mors 8a, 8b, 8b, est prévue une partie de préhension 15 s'effilant en forme de coin vers l'extrémité libre du mors 8a, 8b, 8b, et facilitant la traction de la verge lors de l'utilisation de l'applicateur. Par ailleurs, l'applicateur comprend, au voisinage des extrémités libres des mors, une bague 16, qui peut être désolidarisée des mors lorsque ceux-ci sont pressés les uns vers les autres, et qui comporte une gorge de réception 17 du manchon 4 en position enroulée, ainsi qu'un rebord d'appui 18 du manchon facilitant son déroulement.

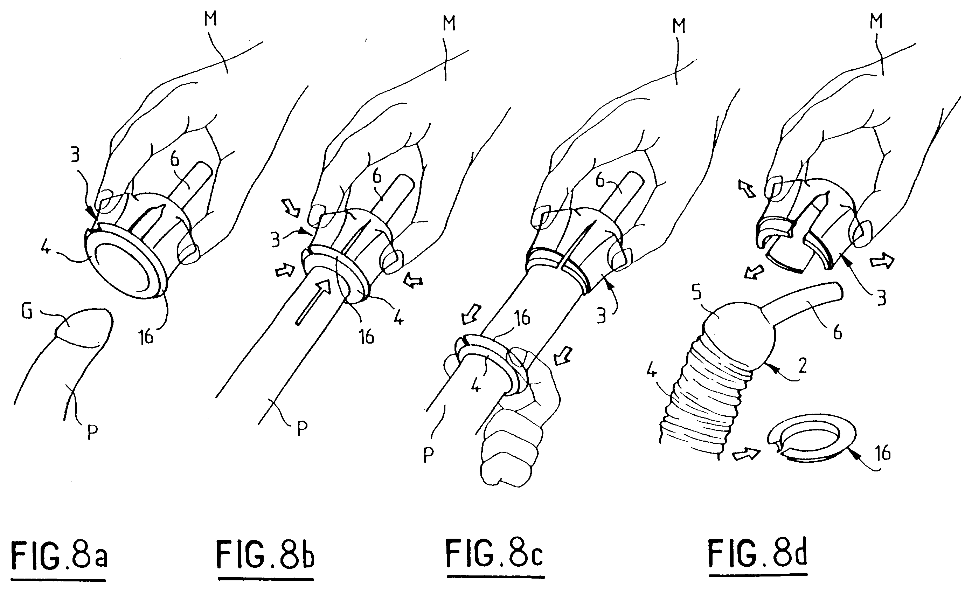

La mise en place du cathéter externe de collecte urinaire, dans cet exemple de réalisation du dispositif, est tout à fait analogue au premier exemple de réalisation, et est illustrée par les figures 8a-8d. On notera toutefois que, dans ce cas, la bague 16, désolidarisée des mors de l'applicateur 3 lorsque ceux-ci sont pressés, est utilisée pour le déroulement du manchon 4 (figure 8c).

## Revendications

1. Applicateur pour cathéter externe de collecte urinaire, ledit cathéter (2) comprenant un manchon souple (4) destiné à être appliqué de façon étanche sur le pénis d'un patient, lequel manchon (4) est raccordé à une partie de réception du gland (5), de forme tubulaire, elle-même raccordée à un tube (6) d'évacuation de l'urine, ledit manchon (4) étant enroulé sur lui-même préalablement à son application, et ledit applicateur (3) comportant un corps tubulaire (7) destiné à entourer au moins partiellement ladite partie de réception du gland (5),
caractérisé en ce que ledit corps (7) comprend au moins deux mors commandables (8a,8b) dont une extrémité de chacun d'entre eux est solidaire d'un support annulaire (9), et dont l'autre extrémité libre présente une saillie (10a,10b) radialement interne destinée à venir s'accrocher derrière le gland et à maintenir celui-ci pendant le déroulement du manchon souple (4) sur le pénis.

2. Applicateur selon la revendication 1,
caractérisé en ce qu'il comprend trois mors (8a,8b,8b), dont un mors central (8a) destiné à venir s'appliquer derrière la face supérieure du gland, et deux mors latéraux (8b,8b), disposés de part et d'autre, respectivement, du mors central (8a).

3. Applicateur selon la revendication 2,
caractérisé en ce que les extrémités libres des deux mors latéraux (8b,8b) sont biseautées de façon correspondant à la forme anatomique des parties latérales du gland derrière lesquelles ils doivent venir s'accrocher.

4. Applicateur selon la revendication 2 ou la revendication 3,
caractérisé en ce que la saillie radialement interne (10a) du mors central (8a) est intérieurement arrondie.

5. Applicateur selon l'une quelconque des revendications 2 à 4,
caractérisé en ce que le corps (7) dudit applicateur (3) présente une forme générale cylindrique, et en ce que des pattes de liaison (11) sont prévues entre chacun desdits mors, reliant ledit support annulaire (9) à une bague (12) munie d'une gorge externe (13) de réception du manchon (4) en position enroulée.

6. Applicateur selon la revendication 5,
caractérisé en ce que les pattes de liaison (11) présentent chacune, au voisinage de ladite bague (12), un épaississement interne (14) dans le prolongement des saillies radialement internes des mors, de façon que l'ensemble saillies-épaississements des pattes présente une forme correspondant à la forme anatomique du gland.

7. Applicateur selon l'une quelconque des revendications 1 à 3,
caractérisé en ce que le corps (7) dudit applicateur (3) présente une forme générale de calotte sphérique, et en ce que, sur la face externe de chacun desdits mors, est prévue une partie de préhension (15) s'effilant en forme de coin vers l'extrémité libre du mors.

8. Applicateur selon la revendication 7,
caractérisé en ce qu'il comprend, au voisinage des extrémités libres des mors, une bague (16), qui peut être désolidarisée desdits mors lorsque ceux-ci sont pressés les uns vers les autres, et qui comporte une gorge (17) de réception du manchon (4) en position enroulée.

9. Applicateur selon la revendication 8,
caractérisé en ce que ladite bague (16) comprend un rebord (18) d'appui du manchon (4) facilitant son déroulement.

10. Dispositif de collecte urinaire, comprenant un cathéther externe (2) comportant un manchon souple (4) destiné à être appliqué de façon étanche sur le pénis d'un patient, lequel manchon (4) est raccordé à une partie de réception du gland (5), de forme tubulaire, elle-même raccordée à un tube (6) d'évacuation de l'urine, ledit manchon (4) étant enroulé sur lui-même préalablement à son application,
caractérisé en ce qu'il comprend un applicateur (3) tel que défini dans l'une quelconque des revendications 1 à 9.

## Claims

1. Applicator for an external urinary collection catheter, the said catheter (2) comprising a flexible sheath (4) intended to be applied in a leaktight manner onto the penis of a patient, which sheath (4) is connected to a part, for receiving the glans (5), of tubular shape, itself connected to a tube (6) for removing urine, the said sheath (4) being rolled on itself prior to its application, and the said applicator (3) comprising a tubular body (7) intended to surround, at least partially, the said part for receiving the glans (5), characterized in that the said body (7) comprises at least two controllable jaws (8a, 8b), one end of each of which is integral with an annular support (9), and the other free end of which has a radially internal projection (10a, 10b) intended to hook behind the glans and to hold the latter during the unrolling of the flexible sheath (4) on the penis.

2. Applicator according to Claim 1, characterized in that it comprises three jaws (8a, 8b, 8b), of which a central jaw (8a) is intended to be applied behind the upper face of the glans, and two lateral jaws (8b, 8b), are arranged on either side, respectively, of the central jaw (8a).

3. Applicator according to Claim 2, characterized in that the free ends of the two lateral jaws (8b, 8b) are bevelled so as to correspond to the anatomical shape of the lateral parts of the glans behind which they are to hook.

4. Applicator according to Claim 2 or Claim 3, characterized in that the radially internal projection (10a) of the central jaw (8a) is internally rounded.

5. Applicator according to any one of Claims 2 to 4, characterized in that the body (7) of the said applicator (3) has a general cylindrical shape, and in that connection tabs (11) are provided between each of the said jaws, joining the said annular support (9) to a ring (12) fitted with an external groove (13) for receiving the sheath (4) in the rolled-up position.

6. Applicator according to Claim 5, characterized in that the connection tabs (11) each have, in the vicinity of the said ring (12), an internal thickening (14) in the extension of the radially internal projections of the jaws, so that the projections/thickenings of the tabs assembly has a shape corresponding to the anatomical shape of the glans.

7. Applicator according to any one of Claims 1 to 3, characterized in that the body (7) of the said applicator (3) has a general shape of a spherical cap, and in that on the external face of each of the said jaws, a gripping part (15) is provided, tapering into the shape of a wedge towards the free end of the jaw.

8. Applicator according to Claim 7, characterized in that it comprises, in the vicinity of the free ends of the jaws, a ring (16), which can be detached from the said jaws when the latter are pressed towards each other, and which comprises a groove (17) for receiving the sheath (4) in the rolled-up position.

9. Applicator according to Claim 8, characterized in that the said ring (16) comprises a lip (18) for supporting the sheath (4) facilitating its unrolling.

10. Urinary collection device comprising an external catheter (2) comprising a flexible sheath (4) intended to be applied in a leaktight manner onto the penis of a patient, which sheath (4) is connected to a part, for receiving the glans (5), of tubular shape, itself connected to a tube (6) for removal of urine, the said sheath (4) being rolled on itself prior to its application, characterized in that it comprises an applicator (3) as defined in any one of Claims 1 to 9.

## Patentansprüche

1. Appliziereinrichtung zum Anbringen eines externen Urinsammelkatheters, wobei der Katheter (2) eine elastische Manschette (4) umfaßt, die dazu bestimmt ist, auf dem Penis eines Patienten dicht angebracht zu werden, wobei die Manschette (4) mit einem röhrenförmig ausgebildeten Teil zur Aufnahme der Eichel (5) verbunden ist, der selbst mit einer Röhre (6) zum Abführen des Urins verbunden ist, wobei die Manschette (4) vor ihrer Anbringung auf sich selbst aufgerollt ist, und wobei die Appliziereinrichtung (3) einen röhrenförmigen Körper (7) besitzt, der dazu bestimmt ist, den Teil zur Aufnahme der Eichel (5) wenigstens teilweise zu umgeben, dadurch gekennzeichnet, daß der Körper (7) mindestens zwei betätigbare Backen (8a, 8b) umfaßt, wovon ein Ende jeder von ihnen mit einem ringförmigen Halter (9) fest verbunden ist und deren anderes freies Ende einen radialen inneren Absatz (10a, 10b) aufweist, der dazu bestimmt ist, sich hinter der Eichel festzuhaken und diese während des Entrollens der elastischen Manschette (4) auf dem Penis festzuhalten.

2. Appliziereinrichtung nach Anspruch 1, dadurch gekennzeichnet, daß sie drei Backen (8a, 8b, 8b) umfaßt, wovon eine mittlere Backe (8a) dazu bestimmt ist, sich hinter der Oberseite der Eichel anzulegen, und zwei seitliche Backen (8b, 8b) zu beiden Seiten der mittleren Backe (8a) angeordnet sind.

3. Appliziereinrichtung nach Anspruch 2, dadurch gekennzeichnet, daß die freien Enden der beiden seitlichen Backen (8b, 8b) entsprechend der anatomischen Form der seitlichen Bereiche der Eichel abgeschrägt sind, hinter denen sie sich festhaken sollen.

4. Appliziereinrichtung nach Anspruch 2 oder Anspruch 3, dadurch gekennzeichnet, daß der radiale innere Absatz (10a) der mittleren Backe (8a) innen abgerundet ist.

5. Appliziereinrichtung nach einem der Ansprüche 2 bis 4, dadurch gekennzeichnet, daß der Körper (7) der Appliziereinrichtung (3) eine allgemein zylindrische Form aufweist, und daß Verbindungsstücke (11) zwischen jeder der Backen vorgesehen sind, die den ringförmigen Halter (9) mit einem Ring (12) verbinden, der mit einer äußeren Rille (13) zur Aufnahme der Manschette (4) in aufgerolltem Zustand versehen ist.

6. Appliziereinrichtung nach Anspruch 5, dadurch gekennzeichnet, daß die Verbindungsstücke (11) jeweils nahe dem Ring (12) eine innere Verdickung (14) in der Verlängerung der radialen inneren Absätze der Backen aufweisen, so daß die Anordnung der Stücke mit Absätzen und Verdickungen eine der anatomischen Form der Eichel entsprechende Form zeigt.

7. Appliziereinrichtung nach einem der Ansprüche 1 bis 3, dadurch gekennzeichnet, daß der Körper (7) der Appliziereinrichtung (3) die allgemeine Form einer runden Kappe aufweist und daß auf der Außenseite jeder der Backen ein Greifteil (15) vorgesehen ist, der sich zum freien Ende der Backe hin keilförmig verjüngt.

8. Appliziereinrichtung nach Anspruch 7, dadurch gekennzeichnet, daß sie in der Nähe der freien Enden der Backen einen Ring (16) umfaßt, der von den Backen getrennt werden kann, wenn diese zueinander gedrückt werden, und der eine Nut (17) zur Aufnahme der Manschette (4) in aufgerolltem Zustand aufweist.

9. Appliziereinrichtung nach Anspruch 8, dadurch gekennzeichnet, daß der Ring (16) einen Rand (18) zum Halten der Manschette (4) umfaßt, der ihr Entrollen erleichtert.

10. Urinsammelvorrichtung, die einen externen Katheter (2) umfaßt, der eine elastische Manschette (4) besitzt, die dazu bestimmt ist, an dem Penis eines Patienten dicht angebracht zu werden, wobei die Manschette (4) mit einem röhrenförmig ausgebildeten Teil zur Aufnahme der Eichel (5) verbunden ist, der selbst mit einer Röhre (6) zum Abführen des Urins verbunden ist, wobei die Manschette (4) vor ihrer Anbringung auf sich selbst aufgerollt ist, dadurch gekennzeichnet, daß sie eine Appliziereinrichtung (3) umfaßt, wie sie in einem der Ansprüche 1 bis 9 definiert ist.
